# FASCICULE DE BREVET EUROPEEN

(11) **EP 0 950 654 B1**
(45) Date de publication et mention de la délivrance du brevet: **30.07.2003**
(21) Numéro de dépôt: 99400977.7
(22) Date de dépôt: 20.04.1999
(51) Int. Cl.: C07C 209/84

(54) **Procédé de purification de polyamines aromatiques**
Verfahren zur Reinigung aromatischer Polyamide
Process for the purification of aromatic polyamides

(43) Date de publication de la demande: 20.10.1999
(73) Titulaire: RHODIA CHIMIE, 92408 Courbevoie Cédex (FR)
(72) Inventeur: Marion, Philippe, 69390 Vernaison (FR); Montarsolo, Stéphane, 75014 Paris (FR)
(74) Mandataire: Wattremez, Catherine

(56) Documents cités:
- WO-A-94/06752
- DE-C- 19 608 443
- US-A- 3 420 752

## Description

La présente invention a pour objet un procédé de purification de polyamines aromatiques obtenues par hydrogénation des composés polynitrés aromatiques correspondants.

Il est bien connu que les produits issus de la réaction d'hydrogénation de composés polynitrés aromatiques comprennent un mélange des différents isomères des composés polyaminés, ainsi que des sous-produits volatils et des sous-produits dont le point d'ébullition est élevé (appelés aussi par la suite sous-produits lourds).

Ainsi, il est nécessaire, d'une part, et cela de manière évidente, de séparer les composés polyaminés aromatiques des sous-produits, mais d'autre part, il peut s'avérer important de séparer certains isomères de ces polyamines des autres, car tous ne représentent pas le même intérêt pour des applications ultérieures.

On se trouve dans une telle situation lors de la synthèse de la toluène diamine (TDA), qui comprend deux types d'isomères, ortho et méta, dont seuls les deux isomères méta présentent un intérêt, essentiellement pour l'obtention de toluène diisocyanate. En outre, le mélange réactionnel issu de la réaction d'hydrogénation comprend des sous-produits volatils, tels que la toluidine par exemple, ainsi que des sous-produits lourds.

Il est connu dans la technique de traiter le mélange réactionnel issu de la réaction d'hydrogénation des composés dinitrés aromatiques correspondants, par distillation, après avoir mis en oeuvre des étapes préalables de déshydratation du mélange réactionnel et éventuellement d'élimination du solvant s'il était présent lors de la réaction d'hydrogénation. En tête de cette colonne de distillation sont récupérés les sous-produits volatils ainsi que les isomères ortho-TDA (2.3-TDA, 3.4-TDA) ; en pied de colonne, sont récupérés les sous-produits lourds comprenant de la méta-TDA. La quantité de sous-produits lourds peut représenter de 0,1 à 2,5 % en poids du mélange récupéré.

Le mélange des sous-produits lourds et de la méta-TDA est alors traité de manière à vaporiser toute la méta-TDA présente. Cependant, ce procédé est la cause d'une perte en méta-TDA qui se trouve emprisonnée dans les sous-produits lourds. Cette quantité peut représenter 25 à 200 % en poids des composés lourds.

Le problème qui n'est donc pas résolu de manière parfaitement satisfaisante par les procédés classiques, est donc de séparer les isomères méta-TDA de ces sous-produits lourds en perdant le minimum possible de ces isomères.

Dans le but de limiter les pertes en ce composé, la demande de brevet WO 94/006752 a proposé de séparer la méta-TDA des lourds en distillant ce mélange en présence d'un tiers solvant dont le point d'ébullition est supérieur à 290°C. Si ce procédé permet de limiter les pertes en produit désiré dans les lourds, il nécessite cependant la mise en oeuvre d'un tiers composé ce qui augmente les coûts ainsi que l'installation de nouveaux appareillages.

Dans le brevet allemand DE 196 08 443, la solution préconisée consiste en un agencement comprenant la colonne classique de distillation sous vide et des évaporateurs. Plus particulièrement, le mélange de polyamines est introduit dans la colonne de distillation sous vide de laquelle on récupère en tête, un mélange de sous-produits légers ainsi que les isomères de l'ortho-TDA. En pied de colonne, la totalité du flux, comprenant la méta-TDA ainsi que les lourds, est envoyée dans un évaporateur où elle est vaporisée. La fraction vaporisée est condensée et stockée ou mise en jeu dans une réaction chimique ultérieure. La fraction non vaporisée est mélangée à un flux soutiré latéralement dans la moitié supérieure de la colonne de distillation, puis vaporisée à son tour. Les produits restant liquides sont écartés, pour être détruits par exemple, quant à la fraction vaporisée, elle est retournée à la colonne de distillation. Une variante de cet agencement consiste à alimenter la colonne de distillation, non pas avec le mélange TDA et sous-produits, mais avec la fraction vaporisée de ce mélange. La fraction non vaporisée est traitée de la même façon que le flux non vaporisé recueilli après la vaporisation du flux récupéré en pied de colonne de la variante précédente. La méta-TDA purifiée est alors récupérée en pied de la colonne de distillation.

Ce document décrit un procédé permettant de diminuer, sans toutefois les éliminer, les pertes en méta-TDA présente dans les sous-produits lourds. Cependant, ce procédé est complexe et nécessite l'installation de nombreux appareillages.

La présente invention a pour but de proposer un procédé simple et efficace de purification de mélange de polyamines aromatiques, ne nécessitant pas la mise en place de coûteux et nombreux appareils, sans perte en polyamines aromatiques.

Ces buts et d'autres sont atteints par la présente invention qui a donc pour objet un procédé de purification d'un mélange de polyamines aromatiques obtenues par hydrogénation des composés polynitrés aromatiques correspondants, mélange comprenant les différents isomères desdites amines, ainsi que des sous-produits de réaction. Le procédé selon l'invention consiste à effectuer une distillation dudit mélange, puis à opérer de la manière suivante :
- on sépare un premier flux correspondant à une fraction ou à la totalité du flux récupéré en pied de la colonne de distillation, et on l'engage dans une réaction chimique (telle que la phosgénation par exemple),
- on sépare un second flux correspondant :
   (i) à un flux soutiré latéralement en phase gazeuse, dans le tiers inférieur de ladite colonne ; ce flux représentant au plus 20 % ou plus de 80 % du volume total (premier et second flux) ; ou
   (ii) à une fraction du premier flux, cette fraction représentant 2 à 50 % du volume du premier flux, qui est ensuite vaporisé partiellement.

Le procédé selon la présente invention permet donc d'obtenir des isomères souhaités de polyamines (isomères méta, dans le cas de la TDA), exempts de sous-produits lourds, sans perte en polyamines. En effet, la fraction non purifiée (comprenant les isomères méta et les sous-produits lourds) est avantageusement recyclée dans une réaction ultérieure, telle que la phosgénation.

Notons qu'une telle procédure n'était pas évidente en soi car le mélange "recyclé" doit présenter un rapport approprié en isomères souhaités. Par exemple, dans le cas des isomères méta de la toluènediamine, le rapport isomérique 2.4 / 2.6 doit être compris entre 3,6 et 4,2.

Ainsi, le procédé selon l'invention permet d'obtenir à la fois, et dans des proportions respectives adaptables dans une certaine mesure et selon les besoins du marché par exemple, un mélange comprenant les isomères et les sous-produits lourds, et d'autre part, des isomères exempts de sous-produits lourds (polyamine blanchie).

De plus, le procédé selon l'invention de séparation des isomères souhaités des sous-produits lourds est efficace car la pureté en isomères souhaités est d'au moins 99 %, et de manière avantageuse, supérieure ou égale à 99,5 %.

Ces buts et d'autres apparaîtront plus clairement à la lecture de la description et des figures annexées qui vont suivre.

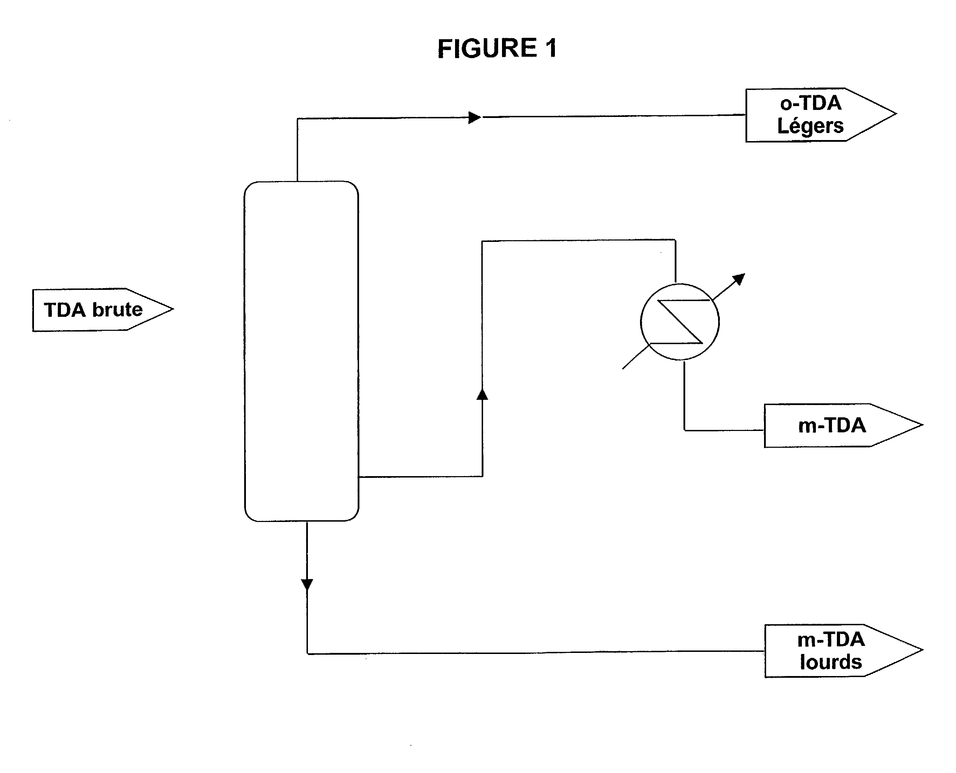

La figure 1 représente une première variante, dans laquelle un flux latéral gazeux (correspondant au second flux(i)) est soutiré de la colonne de distillation puis introduit dans un condenseur, de manière à obtenir les isomères méta souhaités exempts de sous-produits lourds. En pied de cette colonne, on récupère un flux (correspondant au premier flux) comprenant les mêmes isomères souhaités avec les sous-produits lourds. De manière avantageuse, ce flux est engagé sans autre traitement de purification dans une réaction chimique.

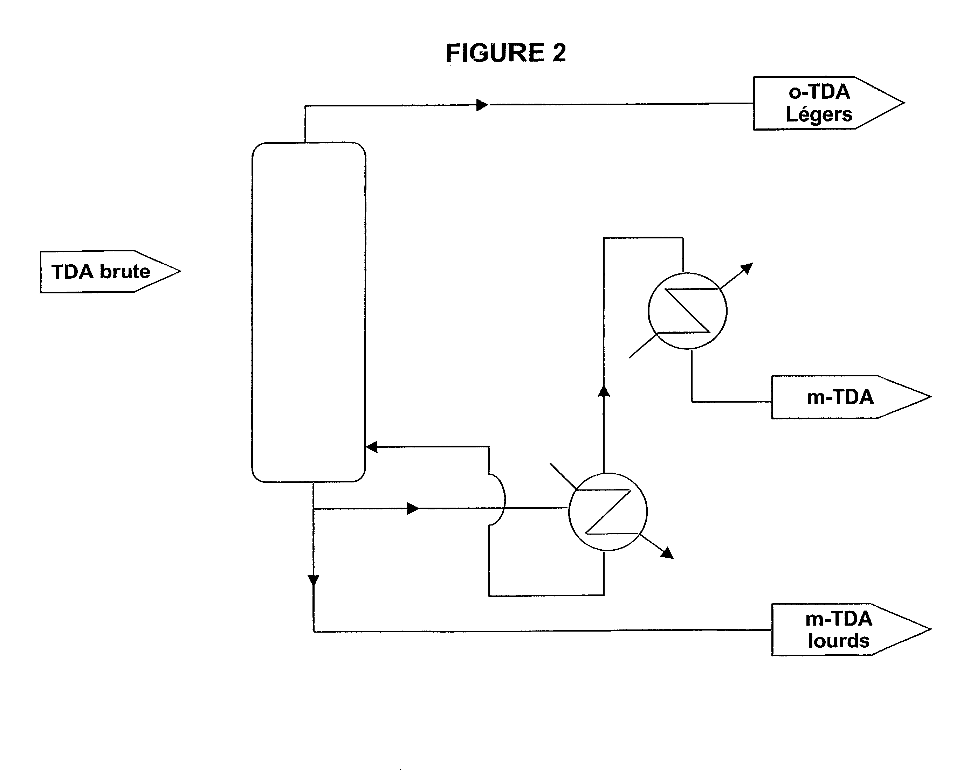

La figure 2 représente la seconde variante dans laquelle une fraction du premier flux (correspondant au second flux (ii)) est envoyée dans un évaporateur afin d'y être partiellement vaporisé. La fraction restant liquide est retournée à la colonne de distillation, au niveau du quart inférieur de cette dernière. La fraction vaporisée, comprenant l'isomère souhaité, est condensée et stockée.

Avant de décrire dans le détail le procédé selon l'invention, les polyamines aromatiques traitées vont être définies.

Ainsi, on entend par polyamine aromatique, tout composé comprenant au moins deux fonctions amine primaire et au moins un motif aromatique en C₆-C₁₄, de préférence en C₆-C₁₀, substitué ou non par un ou plusieurs radicaux hydrocarbonés, saturés ou non, linéaires, cycliques ou ramifiés en C₁-C₁₀.

Plus précisément, les radicaux hydrocarbonés précités, substituant éventuellement lesdits motifs aromatiques, peuvent être choisis parmi les radicaux alkyles, aryles, alkyle-aryles et aryle-alkyles en C₁-C₁₀, de préférence en C₁-C₆.

De préférence, on entend par polyamine aromatique un composé de formule : H₂N-R-NH₂, formule dans laquelle R représente le motif aromatique, substitué ou non, décrit ci-dessus.

Selon un mode plus particulier de réalisation de l'invention, le motif R précité est éventuellement substitué par un ou plusieurs radicaux alkyles en C₁-C₁₀, de préférence en C₁-C₆. A titre d'exemple, on peut notamment citer les noyaux benzénique et naphtalénique substitués ou non par un ou plusieurs radicaux méthyle, éthyle, propyle, butyle, pentyle, hexyle et/ou leurs isomères.

De façon préférée, la polyamine aromatique est choisie parmi la toluène diamine et ses isomères, la xylylène diamine et ses isomères, la phénylène diamine et ses isomères.

Ainsi que cela été indiqué auparavant, lesdites polyamines aromatiques sont obtenues par hydrogénation des composés polynitrés aromatiques correspondants, dont la formule est la suivante : O₂N-R-NO₂, formule dans laquelle R a la même définition que précédemment.

L'hydrogénation des composés nitrés est tout à fait classique dans le domaine.

Elle est réalisée en mettant en oeuvre un catalyseur, qui se trouve dispersé dans le mélange réactionnel. Ce dernier est séparé en fin de réaction par filtration, notamment.

Il est à noter que la réaction d'hydrogénation peut ou non être mise en oeuvre en présence d'un solvant inerte dans les conditions de réaction. Parmi les solvants classiques, on peut citer les alcools présentant un point d'ébullition faible, tels que le méthanol, l'éthanol, l'isopropanol, ou même encore des éthers, tels que le tétrahydrofuranne.

Le mélange réactionnel issu de l'hydrogénation comprend les différents isomères des composés polyaminés, des sous-produits lourds et volatils ainsi que de l'eau, et le cas échéant, le solvant.

Classiquement, on met en oeuvre une étape de séparation préliminaire du solvant dans le cas où ce dernier est présent, puis de l'eau produite lors de la réaction.

Ces opérations sont tout à fait habituelles dans le domaine et sont réalisées en général par distillation du mélange réactionnel.

Dans tout ce qui va suivre, et pour des raisons de simplificatior de l'exposé, il ne sera fait référence qu'à la toluène diamine, à ses isomères ortho et méta et aux sous-produits lourds et volatils, obtenus lors de l'hydrogénation des dinitrotoluènes, sachant que le procédé selon l'invention peut être mis en oeuvre pour la purification d'autres mélanges de polyamines obtenues par hydrogénation des composés polynitrés correspondants.

Une fois cette opération réalisée, le mélange réactionnel est alimenté dans une colonne de distillation. Cette colonne est habituellement utilisée dans les procédés de purification des polyamines aromatiques, et a pour but de séparer les isomères souhaités de ceux qui présentent peu d'intérêt et des sous-produits lourds et volatils. Ainsi, et dans le cas particulier de la TDA, en tête de ladite colonne sont récupérés les sous-produits volatils avec l'isomère ortho, en pied, sont récupérés les sous-produits lourds avec de la méta-TDA.

Cette colonne comprend plus particulièrement de 30 à 60 étages théoriques.

Elle peut comprendre indifféremment un garnissage ordonné, des anneaux, des plateaux (perforés, clapets, cloches, etc.).

Généralement, la distillation est effectuée sous vide.

Il est à noter que les paramètres de conduite de la colonne de distillation dépendent bien évidemment de la nature du mélange à traiter.

A titre d'exemple, la distillation du mélange comprenant les divers isomères de la TDA et les sous-produits qui l'accompagnent, est réalisée de telle sorte que la température en pied de la colonne soit de l'ordre de 180 à 220°C.

Quant à la pression en tête de colonne, toujours dans le cas des mélanges précités comprenant la TDA, celle-ci est par exemple de l'ordre de 3 à 120 kPa.

Le mélange à traiter est alimenté, de manière avantageuse, sous la forme d'un flux liquide.

Le niveau de l'introduction du flux dans la colonne peut être déterminé de manière habituelle par l'homme de l'art. Cependant, à titre d'exemple, ce flux est alimenté dans le deuxième tiers de la colonne.

En tête de cette colonne, on récupère classiquement, les sous-produits volatils ainsi que les isomères dont les points d'ébullition sont les plus faibles (ortho-TDA).

En pied de colonne on récupère un flux liquide, appelé premier flux, qui comprend les sous-produits lourds avec les isomères dont les points d'ébullition sont les plus élevés (soit les méta-TDA).

De manière avantageuse, ce premier flux est directement engagé, c'est-à-dire sans subir d'autre étape de purification, dans une réaction chimique ultérieure comme la phosgénation par exemple.

La première variante selon l'invention consiste à soutirer latéralement dans le tiers inférieur de la colonne, un flux (appelé second flux (i)), qui se présente plus particulièrement sous forme gazeuse. De préférence, le prélèvement a lieu au dessus du bouilleur, et plus particulièrement 1 à 3 plateaux au dessus du bouilleur.

Ce second flux (i) comprend les isomères souhaités, exempts de sous-produits lourds. Il est de manière avantageuse simplement condensé dans un appareil approprié, puis si nécessaire, stocké.

Selon cette variante, ce second flux (i) représente au plus 20 % du volume total, ou plus de 80 % du volume total. Notons que par volume total, dans le cas de la première variante, on entend désigner le flux constitué de celui récupéré en pied de colonne (premier flux) et celui soutiré latéralement (second flux (i)).

Plus particulièrement, le second flux (i) représente de 0 exclu à 20 % du volume total, ou bien de 80 à 99 % du volume total.

De préférence, le second flux (i) représente au plus 20 % du volume total, de préférence de 1 à 20 % du volume total.

La seconde variante de l'invention consiste, non plus à soutirer latéralement un flux, mais à écarter une fraction du flux récupéré en pied de colonne (soit le premier flux), et qui contient donc les sous-produits lourds ainsi que la méta-TDA.

Dans le cas de cette seconde variante, la fraction écartée, correspondant au second flux (ii), représente de 2 à 50 % du premier. De manière avantageuse, cette fraction représente 5 à 30 % du premier flux.

Ce second flux (ii) est ensuite vaporisé partiellement.

Selon un mode de réalisation particulier de l'invention, la partie vaporisée représente au plus 97 % en volume total du second flux (ii). De préférence, la partie vaporisée représente entre 50 et 95 % en volume par rapport à la même référence.

Cette opération a lieu dans tout type d'appareillage approprié, tel qu'un bouilleur ou encore un évaporateur à film.

Généralement, cette opération a lieu sous vide. On peut procéder à la vaporisation, sous une pression similaire à celle de la colonne.

Le taux de vaporisation recherché de la méta-TDA n'étant pas maximum, il est à noter que, de manière avantageuse, il n'est pas nécessaire de mettre en oeuvre des pressions très réduites. Ainsi, des pressions de l'ordre de 6 à 100 kPa conviennent.

La température dépendra d'une part des composés à valoriser, de même que du taux de vaporisation souhaité. l'homme de l'art est à même de régler ces conditions de mise en oeuvre.

La fraction vaporisée, qui est exempte de sous-produits lourds, est ensuite condensée avant d'être stockée si nécessaire.

La fraction restant liquide est retournée à la colonne de distillation.

Il est à noter que ce flux liquide peut retourner directement à la colonne de distillation. Selon cette possibilité, le flux est introduit dans la colonne dans le tiers inférieur de la colonne, et de préférence au niveau du bouilleur ou bien 1 à 2 étages théoriques au-dessus de ce dernier. C'est cette possibilité qui est représentée sur la figure 2.

Selon une autre possibilité, qui n'est pas représentée sur la figure 2, ce flux liquide est mélangé ou introduit au même niveau que celui introduit initialement dans la colonne, en d'autres termes le mélange réactionnel issu de l'hydrogénation, ayant subi préalablement, une déshydratation et éventuellement une élimination du solvant s'il était présent lors de la réaction.

Il est de même possible de mélanger ce flux liquide à celui récupéré en pied de colonne après le fractionnement donnant le second flux (ii).

## Revendications

1. Procédé de purification d'un mélange de polyamines aromatiques obtenues par hydrogénation des composés polynitrés aromatiques correspondants, mélange comprenant les différents isomères desdites amines, ainsi que des sous-produits de réaction ; purification dans laquelle on effectue une distillation du mélange, **caractérisé en ce que** l'on opère de la manière suivante :
- on sépare un premier flux correspondant à une fraction ou à la totalité du flux récupéré en pied de la colonne de distillation, et on l'engage dans une réaction chimique,
- on sépare un second flux correspondant :
(i) à un flux soutiré latéralement en phase gazeuse, dans le tiers inférieur de ladite colonne ; ce flux représentant au plus 20 % ou plus de 80 % du volume volume total (premier et second flux) ;ou
(ii) à une fraction du premier flux, cette fraction représentant 2 à 50 % du volume du premier flux, qui est ensuite vaporisé partiellement.

2. Procédé selon la revendication précédente, **caractérisé en ce que** le second flux (i) correspondant à un flux soutiré latéralement, représente de 0 exclu à 20 % du volume total (premier et second flux), ou de 80 à 99 % du volume total (premier et second flux).

3. Procédé selon la revendication précédente, **caractérisé en ce que** le second flux (i) est condensé.

4. Procédé selon la revendication 1, **caractérisé en ce que** le second flux (ii), représente 5 à 30 % du volume du premier flux.

5. Procédé selon l'une des revendications 1 ou 4, **caractérisé en ce que** la partie vaporisée représente au plus 97 % en volume total du second flux (ii), et de préférence représente entre 50 et 95 % en volume par rapport à la même référence.

6. Procédé selon l'une quelconque des revendications 4 ou 5, **caractérisé en ce que** la vaporisation a lieu sous une pression comprise entre 6 et 100 kPa.

7. Procédé selon l'une quelconque des revendications 4 à 6, **caractérisé en ce que** la partie non vaporisée de ce second flux (ii) est retournée à la colonne de distillation et que la partie vaporisée est condensée.

## Patentansprüche

1. Verfahren zur Reinigung einer Mischung aromatischer Polyamine, die durch Hydrierung entsprechender polynitrierter aromatischer Verbindungen erhalten werden, wobei die Mischung verschiedene Isomere dieser Amine sowie Reaktionsnebenprodukte enthält, wobei bei der Reinigung eine Destillation der Mischung durchgeführt wird, **dadurch gekennzeichnet, daß** man in folgender Weise verfährt:
- Man trennt einen ersten Fluß ab, der einer Fraktion oder der Gesamtheit des am Fuß der Destillationskolonne erhaltenen Flusses entspricht, und setzt ihn in einer chemischen Reaktion ein,
- man trennt einen zweiten Fluß ab, der
(i) einem Fluß, der seitlich in der Gasphase im unteren Drittel der Kolonne abgezogen wird, wobei dieser Fluß höchstens 20 % oder mehr als 80 % des Gesamtvolumens (erster und zweiter Fluß) darstellt; oder
(ii) einer Fraktion des ersten Flusses, wobei diese Fraktion 2 bis 50 % des Volumens des ersten Flusses darstellt, der anschließend teilweise verdampft wird,
entspricht.

2. Verfahren nach dem vorhergehenden Anspruch, **dadurch gekennzeichnet, daß** der zweite Fluß (i) entsprechend einem seitlich abgezogenen Fluß 0 (ausgeschlossen) bis 20 % des Gesamtvolumens (erster und zweiter Fluß) oder 80 bis 99 % des Gesamtvolumens (erster und zweiter Fluß) darstellt.

3. Verfahren nach dem vorhergehenden Anspruch, **dadurch gekennzeichnet, daß** der zweite Fluß (i) kondensiert wird.

4. Verfahren nach Anspruch 1, **dadurch gekennzeichnet, daß** der zweite Fluß (ii) 5 bis 30 % des Volumens des ersten Flusses darstellt.

5. Verfahren nach einem der Ansprüche 1 bis 4, **dadurch gekennzeichnet, daß** der verdampfte Teil höchstens 97 Vol.-% des Gesamtvolumen des zweiten Flusses (ii) darstellt und vorzugsweise 50 bis 95 Vol.-% in bezug auf dieselbe Bezugsgröße darstellt.

6. Verfahren nach einem der Ansprüche 4 oder 5, **dadurch gekennzeichnet, daß** die Verdampfung unter einem Druck von 6 bis 100 kPa durchgeführt wird.

7. Verfahren nach einem der Ansprüche 4 bis 6, **dadurch gekennzeichnet, daß** der nichtverdampfte Teil des zweiten Flusses (ii) wieder der Destillationskolonne zugeführt wird und daß der verdampfte Teil kondensiert wird.

## Claims

1. Process for the purification of a mixture of aromatic polyamines obtained by hydrogenation of corresponding aromatic polynitrated compounds, mixture comprising the different isomers of said amines, and also the reaction byproducts, purification in which a distillation of the mixture is carried out, **characterized in that** the process is carried out as follows:
- a first flow corresponding to a fraction or to the total flow recovered at the foot of the distillation column is separated, and is used in a chemical reaction,
- a second flow is separated corresponding:
(i) to a flow drawn-off laterally in gaseous phase in the lower third of said column; this flow representing at most 20% or more than 80% of the total volume (first and second flow), or
(ii) to a fraction of the first flow, this fraction representing 2 to 50% of the volume of the first flow, which is then partly vaporized.

2. Process according to the previous claim, **characterized in that** the second flow (i) corresponding to a flow drawn-off laterally represents from 0 (excluded) to 20% of the total volume (first and second flow), or from 80 to 99% of the total volume (first and second flow).

3. Process according to the previous claim, **characterized in that** the second flow (i) is condensed.

4. Process according to claim 1, **characterized in that** the second flow (ii) represents 5 to 30% of the volume of the first flow.

5. Process according to one of claims 1 to 4, **characterized in that** the vapozized part represents at most 97% of the total volume of the second flow (ii), and preferably represents between 50 and 95% by volume relative to the same reference.

6. Process according to any one of claims 4 to 5, **characterized in that** the vaporization takes place under a pressure comprised between 6 and 100 kPa.

7. Process according to any one of claims 4 to 6, **characterized in that** the non-vaporized part of this second flow (ii) is returned to the distillation column and that the vaporized part is condensed.
